(19) Europäisches Patentamt
European Patent Office
Office européen des brevets

(11) Numéro de publication : **0 169 112**
**B1**

(12) **FASCICULE DE BREVET EUROPÉEN**

(45) Date de publication du fascicule du brevet : **26.04.89**

(51) Int. Cl.[4] : **C 07 D233/60**, C 07 D401/12, C 07 D403/12, A 61 K 31/415

(21) Numéro de dépôt : **85401200.2**

(22) Date de dépôt : **18.06.85**

(54) **Imidazolides, procédé d'obtention et application à titre d'intermédiaires de synthèse de conjugués cytotoxiques.**

(30) Priorité : **20.06.84 FR 8409703**
**20.06.84 FR 8409704**

(43) Date de publication de la demande :
**22.01.86 Bulletin 86/04**

(45) Mention de la délivrance du brevet :
**26.04.89 Bulletin 89/17**

(84) Etats contractants désignés :
**AT BE CH DE FR GB IT LI LU NL SE**

(56) Documents cités :
**Arch. Biochem. Biophys. 1959, 82 ,70,77**
**J. Amer. Chem. Soc. 1970,92,76,29-31**
**CA90:164345a**

(73) Titulaire : **SANOFI**
**40, Avenue George V**
**F-75008 Paris (FR)**

(72) Inventeur : **Jansen, Franz K.**
**Chemin des Fresquets Assas**
**F-34160 Castries (FR)**
Inventeur : **Gros, Pierre**
**18 rue des Muriers**
**F-34100 Montpellier (FR)**

(74) Mandataire : **Gillard, Marie-Louise et al**
**Cabinet Beau de Loménie 55, Rue d'Amsterdam**
**F-75008 Paris (FR)**

**EP 0 169 112 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne des nouveaux imidazolides et un procédé pour leur obtention. Les imidazolides selon l'invention conviennent notamment comme agents de couplage de protéines, en particulier pour la synthèse de conjugués cytotoxiques utilisables en thérapeutique.

Les composés selon l'invention répondent à la formule :

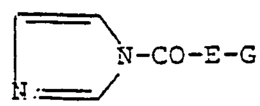

(I)

dans laquelle :

E représente un groupe $-(CH_2)p-$, où p est un nombre entier de 2 à 7 ou un groupe :

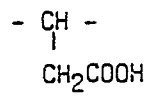

et G est un groupement de structure —S—S—X, où X est un radical activateur choisi parmi les groupes 2-pyridyle et 4-pyridyle non substitués ou substitués par un ou des halogènes ou des radicaux alkyle, carboxyle, alkoxy-carbonyle, le groupe phényle non substitué ou substitué par un ou des halogènes ou des groupes nitro, alkoxy, carboxyle ou alkoxycarbonyle, ou un groupe alkoxycarbonyle.

Les termes « alkyle » et « alkoxy » désignent des groupes contenant jusqu'à 5 atomes de carbone.

Les nouveaux composés de formule I sont préparés par le procédé qui consiste à faire réagir un composé de formule :

G—E—COOH (II)

dans laquelle G et E sont tels que définis ci-dessus, avec le carbonyldiimidazole de formule :

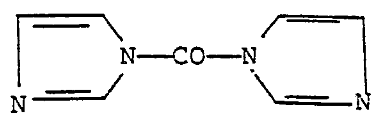

(III)

dans un solvant organique, à la température de 10 à 40 °C. Les solvants éthérés, tels que le dioxanne et le tétrahydrofuranne sont particulièrement préférés.

Les composés de formule I sont particulièrement utiles comme agents de couplage sur les hydroxyles des tyrosines de protéines, telles que les protéines A et P, définies ci-après pour l'obtention des conjugués ou immuno-toxines qui font l'objet de la demande de brevet français déposée ce jour au nom de la demanderesse et intitulée « nouveaux conjugués cytotoxiques utilisables en thérapeutique et procédé d'obtention ».

Ces immunotoxines répondent à la formule statistique suivante :

P'—W—A' (IV)

dans laquelle P' représente le radical d'une protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués, A' représente le radical d'une protéine qui est la sous-unité A de la ricine telle quelle ou chimiquement convenablement modifiée, privée d'au moins une de ses fonctions propres et dans lequel les autres groupes fonctionnels sont éventuellement bloqués et W représente une structure covalente bivalente contenant un groupe disulfure dont les atomes de soufre sont soit ceux des cystéines de P et A, soit sont liés aux fonctions propres de P et/ou de A par des structures d'espacement portant un groupe fonctionnel lié auxdites fonctions propres de P et/ou de A ; avec la limitation que, lorsqu'un des atomes de soufre dudit disulfure est celui de l'une des cystéines de A, l'autre soufre est lié à la protéine P par une structure d'espacement portant un groupe fonctionnel lié à une fonction de la protéine P autre qu'une fonction amine.

Les immunotoxines préférées répondent à la formule statistique :

P'—W'—A' (V)

dans laquelle P' et A' sont tels que définis ci-dessus et W' représente une structure covalente choisie parmi :

(a) un groupement de formule

—Z″—Y—E—S—S—(E′—Y′—Z′)n—

(b) un groupement de formule

—(NH—Y′—E′)n—S—S—E—Y—Z—

où

Z et Z′ représentent les fonctions propres des protéines A et P, choisies parmi l'atome d'oxygène provenant de l'hydroxyle de l'un des résidus de tyrosine ; le groupe carbonyle provenant de l'un des carboxyles terminaux ou des carboxyles libres des acides aspartiques et/ou glutamiques de A et de P ; le groupe provenant de la structure dialdéhydique obtenue après oxydation de la structure glucidique de P par l'acide périodique ; et le groupe —NH— provenant de l'une des amines terminales de A et de P ou de l'une des amines en position epsilon de l'un des résidus de lysine ;

Z″ est tel que défini ci-dessus pour Z et Z′ mais ne peut pas être —NH— ;

Y et Y′ représentent des groupes fonctionnels capables de se lier d'une façon covalente avec l'une quelconque des fonctions Z, Z′ et Z″ des protéines A et P ;

E et E′ représentent des structures d'espacement inertes ; et

n représente zéro ou 1.

Les immunotoxines ci-dessus sont représentées par les formules IV et V en forme simplifiée, mais il est entendu que la structure covalente bivalente —W— ou —W′— est liée à au moins une molécule P et à au moins une molécule A. Le nombre de liaisons avec les protéines P et A dépend du nombre des fonctions propres desdites protéines intervenant dans l'opération de couplage.

Par exemple, si une immunotoxine est formée par couplage de la sous-unité A de la ricine native avec l'anticorps P (par exemple l'anticorps T101) par l'intermédiaire d'une structure covalente bivalente ayant un groupe disulfure dont un soufre est celui de la cystéine 257 de la chaîne A de la ricine et l'autre est lié aux oxygènes phénoliques des tyrosines de l'anticorps P par un groupe oxopropylique, elle aura la formule statistique

P′(O—CO—CH2—CH2—S—S—A′)t

dans laquelle t représente le nombre des tyrosines de l'anticorps (par exemple de l'anticorps T101) intervenues dans le couplage.

L'immunotoxine ainsi obtenue correspond ainsi à un produit de formule V, où :

P′ est tel que défini ci-dessus, notamment le radical de l'anticorps T101 privé de t fonctions phénoliques de ses tyrosines ;

A′ est tel que défini ci-dessus, notamment le radical de la chaîne A de la ricine privée de la fonction thiol de sa cystéine 257 ;

W′ est le groupement (c) :

—Z″—Y—E—S—S—(E′—Y′—Z′)n—

où Z″ est l'oxygène des hydroxyles phénoliques intervenus dans le couplage, Y est —CO— ; E est la structure d'espacement inerte —CH2—CH2— et n est zéro.

Particulièrement préférées sont les immunotoxines formées par une ou plusieurs structures contenant la sous-unité A de la ricine et un seul anticorps P, représentées par la formule statistique

P′(W′—A′)m (VI)

dans laquelle P′, W′ et A′ sont tels que définis ci-dessus et m représente le nombre de fonctions propres de la protéine P intervenues dans le couplage. Le nombre m varie de 0,3 à 12, de préférence de 0,5 à 10.

L'expression « m varie de 0,3 à 12, de préférence de 0,5 à 10 » signifie que la valeur de m est statistique car dans la population des molécules d'anticorps le couplage ne se produit pas d'une façon homogène. Le nombre m peut donc ne pas être entier.

La valeur de m dépend notamment des anticorps utilisés, plus particulièrement de leur poids moléculaire.

Ainsi, si comme anticorps P de départ on utilise un fragment Fab ou Fab′, la valeur de m pourra varier entre 0,3 et environ 2 ; si l'on utilise un fragment F(ab′)2, m pourra varier entre 0,5 et environ 4 ; pour un anticorps du type IgG, la valeur de m sera entre 0,5 et 6 ; enfin, pour un anticorps IgM, la valeur de m pourra varier entre 1 et 12.

Il est cependant préférable que le degré de substitution sur l'anticorps P soit tel qu'il conduise à une valeur de m non inférieure à 0,5 et non supérieure à 10.

Plus généralement, les structures IV et V ci-dessus représentent des formules statistiques écrites de façon simplifiée, comme il a été spécifié ci-dessus.

D'une façon analogue, les formules VII, VIII et XII ci-dessous sont également statistiques — lorsque, le

cas éhéant, n est 1 — car les réactifs de couplage sont préparés à partir de populations de protéines P1 et P2 qui ont toutes exactement les mêmes propriétés que celles prises en compte ci-dessus pour l'anticorps P, que ces protéines P1 et P2 soient elles-mêmes l'anticorps P ou la chaîne A de la ricine.

Les immunotoxines ayant la structure IV ci-dessus peuvent être obtenues par le procédé qui consiste à faire réagir en solution aqueuse et à la température ambiante une protéine P1, qui est indifféremment soit la sous-unité A de la ricine éventuellement modifiée soit un anticorps ou fragment d'anticorps, porteuse d'au moins un groupe thiol libre attaché à ladite protéine P1 directement ou par l'intermédiaire d'une structure d'espacement, avec une protéine P2, différente de P1, qui est indifféremment soit la sous-unité A de la ricine soit un anticorps ou fragment d'anticorps, porteuse d'un groupement capable de couplage avec le thiol libre de la protéine P1, de façon à former une liaison thioéther ou disulfure ; avec la limitation que, dans le cas de la formation de la liaison disulfure, lorsque la protéine P1 est la sous-unité A de la ricine, la liaison avec la protéine P2 est effectuée sur une fonction de ladite protéine P2 autre que les fonctions amine.

Selon un aspect préférentiel, le procédé pour la préparation d'une immunotoxine ayant la structure V, dans laquelle P', W' et A' sont tels que définis ci-dessus, consiste à faire réagir en solution aqueuse et à la température ambiante une protéine de formule

$$P1'—(Z—Y—E)n—SH \qquad (VII)$$

avec une protéine de formule statistique

$$P2'—Z'—Y'—E'—G \qquad (VIII)$$

où P1' et P2' représentent les radicaux des protéines P1 et P2 liés aux fonctions propres desdites protéines ou les radicaux des protéines P1 et P2 provenant de l'ouverture des structures glucidiques par action de l'acide périodique, Z, Z', Y, Y', E et E' sont tels que définis ci-dessus et G représente un groupe —S—S—X, où X est un groupe activateur, étant entendu que lorsque P1' est la sous-unité A de la ricine, n est 1 ou bien n peut être zéro, mais, dans ce cas, Z' est autre que —NH—.

Autant P que A sont donc des protéines qui présentent indifféremment

(1) la ou les fonctions thiol qui participent au couplage et

(2) un ou plusieurs groupements fonctionnels capables de réagir avec les fonctions thiol ci-dessus pour former une liaison disulfure.

Dans la présente description, lesdites fonctions thiol et groupements fonctionnels sont ceux des protéines P ou A natives, ou bien ils y sont introduits artificiellement.

Pour des raisons de clarté on précise ci-après la signification des symboles utilisés pour désigner les protéines ci-dessus ou leurs radicaux et des expressions utilisées pour désigner les différents symboles.

Le symbole P représente une protéine choisie parmi tout anticorps ou fragment d'anticorps, ou toute immunoglobuline ou fragment d'immunoglobuline ou toute molécule dérivant des précédentes par modification artificielle de l'un quelconque de leurs groupements fonctionnels, y compris des structures glucidiques qu'elles portent, sous réserve que la protéine ainsi choisie reste capable de reconnaître sélectivement un antigène donné à la surface des cellules porteuses de cet antigène et notamment des cellules cancéreuses.

Le symbole A représente une protéine qui est la sous-unité dite chaîne A de la toxine végétale ricine telle qu'elle peut être directement obtenue à partir de la ricine naturelle ou toute molécule dérivant de cette chaîne A par modification artificielle de tout groupement fonctionnel porté par cette protéine sous réserve que la protéine ainsi choisie présente encore la propriété d'inhiber la synthèse protéique ribosomale des cellules eucaryotes telle que l'on peut la mettre en évidence dans un modèle d'étude acellulaire.

Le symbole P' représente un radical dérivé de la protéine P ci-dessus telle quelle ou chimiquement convenablement modifiée, privée d'une ou plusieurs fonctions propres et dont d'autres groupes fonctionnels sont éventuellement bloqués.

Le symbole A' représente un radical dérivé de la protéine A ci-dessus telle quelle ou chimiquement convenablement modifiée, privée d'une ou plusieurs fonctions propres et dont d'autres groupes fonctionnels sont éventuellement bloqués.

Le symbole P1 représente une des protéines A et P telles que définies ci-dessus qui porte des fonctions thiol libres, attachées à ladite protéine directement ou par l'intermédiaire d'une structure d'espacement.

Le symbole P2, différent de P1, représente une des protéines A et P telles que définies ci-dessus, qui porte un ou plusieurs groupements fonctionnels capables de réagir avec les thiols libres.

Le symbole P1' représente le radical de la protéine P1 lié aux fonctions propres de la protéine P1 notamment les fonctions SH (de la cystéine), NH2 (terminal de la protéine ou dans la position epsilon des lysines), OH (des tyrosines), COOH (des acides aspartiques et glutamiques) ou le radical de la protéine P1, provenant de l'ouverture des structures glucidiques par action de l'acide périodique lorsque P1 désigne un anticorps ou fragment d'anticorps.

Le symbole P2' représente le radical de la protéine P2 lié aux groupes fonctionnels caractéristiques

NH2 (terminal de la protéine ou dans la position epsilon des lysines), OH (des tyrosines), COOH (des acides aspartiques et glutamiques).

Par exemple, P1'—SH représente la protéine P1 (qui peut être indifféremment l'anticorps ou fragment d'anticorps P ou la sous-unité A de la racine) dans laquelle les groupes SH des cystéines sont libres et les autres groupes fonctionnels sont éventuellement bloqués.

De la même façon, P1'—CO— représente la protéine P1, dans laquelle son groupe carboxylique terminal ou les groupes carboxyliques de ses acides glutamiques et aspartiques sont couplés avec un groupement qui apporte un groupe SH introduit artificiellement.

Encore, P2'—NH— représente la protéine P2 (qui peut être indifféremment l'anticorps ou fragment d'anticorps P ou la sous-unité A de la ricine) dans laquelle son groupe amino terminal ou les groupes amino de ses lysines sont attachés à un groupement susceptible de couplage avec le thiol de la protéine P1.

L'expression « groupe fonctionnel capable de se lier d'une façon covalente » telle qu'utilisée ici pour Y et Y' désigne tous les groupes susceptibles de réagir avec les fonctions propres des protéines P1 et P2 pour donner une liaison covalente. Ainsi, les groupes —CO— et —(C=NH)— sont des groupes fonctionnels convenables susceptibles de se lier aux amines libres, aux thiols et aux hydroxyles phénoliques des protéines. De même, le groupe —NH— est un groupe fonctionnel convenable susceptible de se lier aux groupes carboxyliques libres des protéines. Le groupe =N— est un groupe fonctionnel convenable susceptible de se lier aux deux atomes de carbone des structures glucidiques des protéines P1 ou P2 après oxydation avec les ions periodate lorsque P1 ou P2 désigne un anticorps ou un fragment d'anticorps.

L'expression « fonction propre des protéines » telle qu'utilisée ici pour Z, Z' et Z" désigne les radicaux provenant des groupes caractéristiques des acides aminés formant les protéines P1 et P2, telles que l'atome d'oxygène provenant des hydroxyles des acides aminés tyrosine et éventuellement sérine, le groupe carbonyle provenant du carboxyle terminal ou des carboxyles libres des acides aspartique et glutamique, le groupe —NH— provenant de l'amine terminale des protéines ou des lysines, l'atome de soufre provenant du thiol de la cystéine. La même expression désigne également le groupe provenant de la structure dialdéhydique obtenue après oxydation de la structure gludicique des protéines P1 ou P2 par traitement avec les ions periodate lorsque P1 ou P2 désigne un anticorps ou fragment d'anticorps.

L'obtention de la chaîne A de ricine pure, nécessaire à la réalisation des immunotoxines définies ci-dessus, a été décrite dans le brevet US 4 340 535. La préparation d'anticorps monoclonaux dirigés contre des cellules cancéreuses humaines a été largement mentionnée dans la littérature scientifique et beaucoup de ces anticorps sont maintenant disponibles commercialement.

Le couplage chimique entre la chaîne A de ricine et l'anticorps (ou fragment d'anticorps) peut être réalisé selon des modes opératoires qui :

préservent les activités biologiques respectives des deux composants du conjugué : l'anticorps et la chaîne A de ricine,

assurent au procédé une reproductibilité satisfaisante et un bon rendement de couplage,

permettent de maîtriser la valeur du rapport chaîne A de ricine/anticorps dans le conjugué obtenu,

conduisent à un produit stable et soluble dans l'eau.

Parmi les modes opératoires répondant à ces caractéristiques, il faut privilégier ceux qui mettent en œuvre une ou plusieurs fonctions thiol pour l'établissement de la liaison entre les deux protéines. En effet, ces fonctions thiols se prêtent particulièrement bien à l'établissement de liaisons disulfure qui satisfont aux conditions générales ci-dessus.

D'une façon générale, pour la bonne conduite des réactions de couplage entre protéines et en vue d'éliminer notamment les réticulations anarchiques, il importe que l'une des protéines à coupler et elle seule porte la ou les fonctions thiol à mettre en œuvre, alors que l'autre protéine et elle seule porte une ou plusieurs fonctions susceptibles de réagir avec les thiols en milieu aqueux de pH compris entre 5 et 9 et à une température ne dépassant pas 30 °C, pour fournir une liaison covalente stable et définie.

Les caractéristiques des protéines P1 et P2 utilisées comme produits de départ sont illustrées en détail ci-après. La structure d'espacement E peut être remplacée par les structures préférentielles R à R8 qui ne sont données qu'à titre d'exemple.

I — Cas de la protéine P1

Cette protéine étant dans tous les cas celle qui porte la ou les fonctions thiol qui participeront au couplage, la situation se présente de façon diverse selon la nature de cette protéine P1.

A) La protéine P1 porte naturellement un ou plusieurs radicaux thiol utilisables pour permettre le couplage à la protéine P2 : c'est notamment le cas si la protéine P1 est le fragment d'anticorps connu sous la dénomination de F(ab)', tel qu'il est classiquement obtenu par protéolyse limitée de l'anticorps en présence de pepsine, suivie d'une réduction du (ou des) ponts disulfure entre chaînes lourdes.

C'est également le cas si la protéine P1 est la chaîne A de la ricine, ou un dérivé de cette chaîne A où l'un au moins des groupements thiol portés par les résidus de cystéine 171 et 257 de la chaîne A de ricine native est libre et accessible au couplage chimique.

Dans tous ces cas, la protéine P1 porteuse de son (ou ses) groupement(s) thiol naturels peut être utilisée dans cet état pour l'étape de couplage.

B) La protéine P1 ne porte pas naturellement de radicaux thiol utilisables pour permettre le couplage à la protéine P2 :

— c'est notamment le cas si la protéine P1 est une immunoglobuline native, un anticorps entier ou un fragment d'anticorps et notamment l'un des fragments couramment dénommés F(ab)'2 ou F(ab),

— un autre cas où la protéine P1 ne porte pas naturellement de fonction thiol utilisable pour le couplage est celui où cette protéine P1 est la chaîne A de ricine où chacun des deux résidus de cystéine est soit bloqué par alkylation, soit inaccessible à la modification chimique.

Dans tous les cas, il conviendra alors d'introduire artificiellement sur de telles molécules une ou plusieurs fonctions thiol aptes à permettre le couplage.

Trois types de réaction peuvent être utilisés de préférence pour l'introduction de fonctions thiol :

1 — Le premier type de réaction est l'action de l'anhydride S-acétylmercapto succinique qui est capable d'acyler des fonctions aminées de la protéine. On pourra ensuite libérer les fonctions thiol par élimination du radical acétyl protecteur, par action de l'hydroxylamine, ainsi que cela a été décrit (Archives of Biochemistry and Biophysics, 119, 41-49, 1967). On pourra même, dans le cas où la (ou les) fonctions(s) thiol ainsi introduites sous forme protégée doivent réagir ultérieurement avec un radical disulfure mixte activé, se dispenser de la déprotection préalable par l'hydroxylamine ; en effet, la réaction de formation de la liaison disulfure utilisant les réactifs objet de la présente invention se produit aussi bien avec le radical S-acétyl qu'avec le thiol libre.

D'autres méthodes décrites dans la littérature scientifique peuvent aussi être utilisées pour l'introduction de fonctions thiol sur la protéine à modifier.

2 — Le deuxième type de réaction consiste à faire réagir la protéine par ses groupements carboxyliques avec une molécule diaminée symétrique présentant un pont disulfure, de formule :

H2N—R1—S—S—R1—NH2

dans laquelle R1 est un groupe aliphatique comportant de 2 à 5 atomes de carbone.

La réaction se fait de préférence avec la cystamine [R1 = —(CH2)2—] en présence d'un agent de couplage tel qu'un carbodiimide et notamment un dérivé soluble dans l'eau comme l'éthyl-1 (diméthylamino-3 propyl)-3 carbodiimide, et conduit à la formation, selon les stoechiométries mises en œuvre, de l'un des dérivés suivants ou du mélange des deux :

P1'—CO—NH—R1—S—S—R1—NH2 (IVa)

P1'—CO—NH—R1—S—S—R1—NH—CO—P1 (IVb)

Un tel produit de réaction peut être alors utilisé de deux manières :

a) Si dans les formules Ia ou Ib, la protéine P1 est la chaîne A de ricine ou l'un de ses dérivés le milieu réactionnel obtenu est soumis sans fractionnement à l'action d'un réducteur tel que le mercapto-2-éthanol, ce qui conduit à l'obtention d'un dérivé protéique unique de formule générale :

P1'—CONH—R1—SH

Le produit ainsi obtenu est alors purifié par dialyse ou filtration sur gel.

b) Si dans les formules IVa et IVb le radical P1' est le radical de la protéine P1 constituée par un anticorps ou l'un de ses fragments, le milieu réactionnel obtenu sera utilisé tel quel pour le couplage en utilisant alors une méthode d'échange thiol/disulfure par exemple celle décrite par Cilliland et Collier (Cancer Research, 40, 3564, 1980).

3 — Le troisième type de réaction consiste à utiliser des motifs glucidiques naturellement présents sur les anticorps pour fixer le radical porteur du thiol que l'on se propose d'introduire. La protéine est alors soumise à une oxydation par les ions periodate afin de faire apparaître des fonctions aldéhyde sur les motifs glucidiques. Après arrêt de la réaction par addition d'un excès d'éthylène glycol et élimination des sous-produits et excès de réactifs par dialyse, le produit obtenu est traité par une molécule diaminée symétrique présentant un pont disulfure, de formule générale

H2N—R1—S—S—R1—NH2

dans laquelle R1 est un groupe aliphatique comportant de 2 à 5 atomes de carbone. Les produits d'addition formés sont alors réduits en amines secondaires ou tertiaires par action d'un hydrure

métallique convenable, notamment le borohydrure de sodium. La réaction se fait de préférence avec la cystamine [R1 = —(CH2)2—] et conduit à la formation, selon les stoechiométries mises en œuvre de l'un des dérivés suivants ou du mélange des deux :

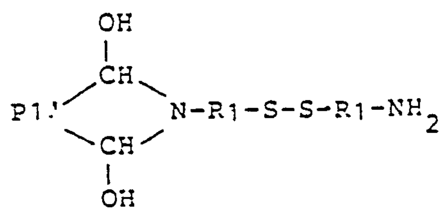

(Va)

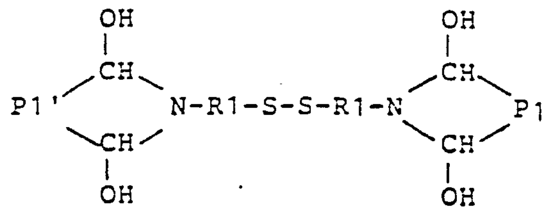

(Vb)

Le milieu réactionnel obtenu pourra alors être traité exactement comme indiqué ci-dessus pour les produits caractérisés par les structures IVa ou IVb où P'1 représente un anticorps ou fragment d'anticorps.

Dans les deux derniers types de réaction d'introduction artificielle de groupements thiols décrits ci-dessus (ceux utilisant un réactif disulfure diaminé symétrique), il est préférable que la protéine P1 mise en œuvre ne possède ni groupes SH libres, ni groupes aminés libres.

Dans le cas de la chaîne A et de ses dérivés cela peut toujours être réalisé par alkylation du ou des SH naturels obtenue par réaction avec un réactif usuel des thiols tel que le N-éthylmaléiimide ou l'acide iodacétique ou l'un de ses dérivés et par méthylation des NH2 naturels selon le procédé de méthylation réductive décrit par MEANS et FEENEY (Biochemistry 7, 2192 (1968)). On peut introduire ainsi dans la chaîne A de ricine native jusqu'à 6 radicaux méthyle par mole. La protéine ainsi modifiée conserve l'intégralité de ses propriétés biologiques et notamment sa capacité à inhiber la synthèse protéique ribosomale dans les cellules eucaryotes.

Dans les cas des anticorps ou fragments d'anticorps et plus généralement de toutes les substances du premier groupe tel que défini précédemment qui ne possèdent pas de groupements SH naturellement libres, il conviendra de procéder à une méthylation réductive, par exemple selon la méthode de MEANS et FEENEY : on peut ainsi habituellement introduire plusieurs dizaines de radicaux méthyle par mole d'anticorps sans modifier sa capacité à reconnaître sélectivement un antigène à la surface des cellules porteuses de cet antigène.

II — Cas de la protéine P2

Cette protéine est dans tous les cas celle qui porte un ou plusieurs groupements fonctionnels capables de réagir avec les thiols de la protéine P1 pour former une liaison disulfure. Ces groupements fonctionnels, toujours artificiellement introduits sur la protéine P2 sont choisis comme indiqué ci-après.

La préparation du conjugué peut alors être représenté par le schéma :

P1'—(Z—Y—E)n—SH + P2'—Z'—Y'—E'—S—S—X ⟶

P1'—(Z—Y—E)n—S—S—E'—Y'—Z'—P2' + X—SH

La protéine P2 substituée par un atome de soufre active est obtenue à partir de la protéine P2 elle-même ou de la protéine P2 correctement protégée, par substitution à l'aide d'un réactif lui-même porteur d'un atome de soufre activé selon le schéma :

P2 + L—Y'—R—S—S—X ⟶ P2'—Z'—Y'—R'—S—S—X

dans lequel :

P2 désigne la protéine à substituer

L—Y' représente une fonction permettant la fixation covalente du réactif sur la protéine.

Le groupement fonctionnel L—Y' est une fonction capable de se lier de façon covalente avec l'une quelconque des fonctions portées par les chaînes latérales des aminoacides constitutifs de la protéine à substituer. Parmi celles-ci, on citera notamment les fonctions phénols des radicaux tyrosyle contenus

dans la protéine. Dans ce cas L—Y' pourra notamment représenter un groupement imidazolyl-l-carbonyl, réagissant avec les groupes phénol de la protéine selon la réaction :

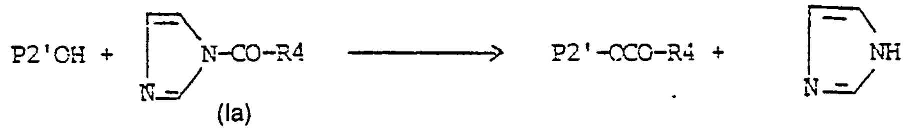

(Ia)

où le 1-imidazolyle est L, le groupe CO est Y' et R4 est le groupe —R—S—S—X. Le composé de formule Ia est un imidazolide selon l'invention. Le radical —S—S—X désigne un disulfure mixte activé capable de réagir avec un radical thiol libre. En particulier, dans ce disulfure mixte, X pourra désigner un groupe pyridyl-2 ou pyridyl-4, éventuellement substitué par un ou des radicaux alkyle, halogène ou carboxylique. X peut aussi désigner un groupe phényle de préférence substitué par un ou des groupes nitro- ou carboxylique. X peut encore représenter un groupe alcoxycarbonyle tel que le groupe méthoxycarbonyle.

Le radical R désigne la structure d'espacement (indiquée comme E dans la formule générale II ci-dessus) capable de porter simultanément les substituants Y' et S—S—X. Il devra être choisi de façon à ne pas comporter de fonctions susceptibles d'interférer au cours des réactions ultérieures avec les réactifs utilisés et les produits synthétisés. En particulier, le groupe R peut être un groupe $—(CH2)n—$ avec n compris entre 1 et 10, ou encore un groupe :

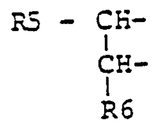

dans lequel R6 désigne l'hydrogène ou un groupe alkyle ayant de 1 à 8 atomes de carbone et R5 désigne un substituant inerte vis-à-vis des réactifs utilisés ultérieurement tel qu'un groupe carbamate

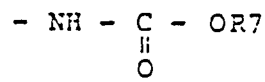

où R7 désigne un groupe alkyle droit ou ramifié ayant de 1 à 5 atomes de carbone et notamment le groupe tertiobutyle. La réaction du comoosé L—Y'—R—S—S—X avec la protéine P2 est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20 % en volume lorsqu'il s'agit d'un alcool tertiaire tel que le butanol tertiaire ou 10 % en volume lorsqu'il s'agit du diméthylformamide ou du tétrahydrofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine P1 est réalisé par mise en présence des deux protéines en solution aqueuse de pH compris entre 6 et 8, à une température ne dépassant pas 30 °C, pendant un temps variant de 1 heure à 24 heures. La solution aqueuse obtenue est éventuellement dialysée pour éliminer les produits de faible masse moléculaire, puis le conjugué peut être purifié par diverses méthodes connues.

Ainsi, les imidazolides selon l'invention conviennent comme agent de couplage des protéines P1 et P2. Ils permettent l'obtention des nouveaux produits ayant la formule statistique :

$$P2''—O—CO—E—G \qquad (IX)$$

dans laquelle P"2 représente le radical d'une protéine choisie parmi

(a) tout anticorps ou fragment d'anticorps ou toute immunoglobuline ou fragment d'immunoglobuline ou toute molécule dérivant des précédentes par modification artificielle de l'un quelconque de leurs groupements fonctionnels ; et

(b) la sous-unité A de la ricine ou toute molécule dérivant de ladite sous-unité A par modification artificielle de l'un quelconque de leurs groupements fonctionnels ;

ledit radical étant privé d'un ou plusieurs groupes hydroxyles phénoliques des tyrosines

— l'atome d'oxygène est celui des groupes hydroxyles phénoliques manquant dans le radical P2" ;

— E et G sont tels que définis ci-dessus.

Les produits de formule IX sont préparés en faisant réagir un produit de formule

$$P2''—OH$$

dans laquelle P2" est tel que défini ci-dessus et le groupe hydroxyle est l'hydroxyle phénolique manquant dans les tyrosines du radical P2", avec un composé de formule I ci-dessus à une température de 10 à 40 °C dans un solvant aqueux contenant éventuellement un solvant organique miscible à l'eau, tel que par exemple un solvant éthéré comme le dioxanne ou le tétrahydrofuranne.

Les exemples qui suivent permettent de mieux comprendre l'invention sans en limiter la portée.

Dans tous ces exemples, la préparation des conjugués sera décrite à partir de la chaîne A de ricine sous sa forme native, telle qu'elle est obtenue par le procédé décrit dans le brevet US 4 340 535.

Les anticorps utilisés dans ces exemples sont :

soit l'anticorps monoclonal T101 dirigé contre l'antigène T65 présent sur les lymphocytes T humains et de nombreuses lignées de cellules de leucémies T humaines. Cet anticorps est celui décrit dans Journal of Immunology 125 (2), 725-7 (1980). Il est commercialement disponible auprès de HYBRITECH Inc. SAN DIEGO, California, USA

soit l'anticorps monoclonal AT15E, dirigé contre l'antigène Thy 1.2 des lymphocytes murins. Cet anticorps est celui décrit dans Journal of Immunology 122, 2491-8 (1979) et a été obtenu à partir de l'hybridome décrit dans Hybridoma 1 (1) 13-17 (1981)

soit un anticorps monoclonal anti-DNP.

Exemple 1

P1 = anticorps T101 sur lequel on a introduit un SH par l'intermédiaire des amines.

P2 = Chaîne A de ricine sur laquelle on a introduit un groupement disulfure activé par l'intermédiaire de l'hydroxyle des tyrosines.

A) Préparation du réactif de couplage

Il s'agit de l'imidazolide dérivé de l'acide (pyridyl-2 disulfanyl)-3 propionique (APDP). Cet imidazolide s'obtient en une seule étape à partir de l'APDP et du carbonyl diimidazole (CDI).

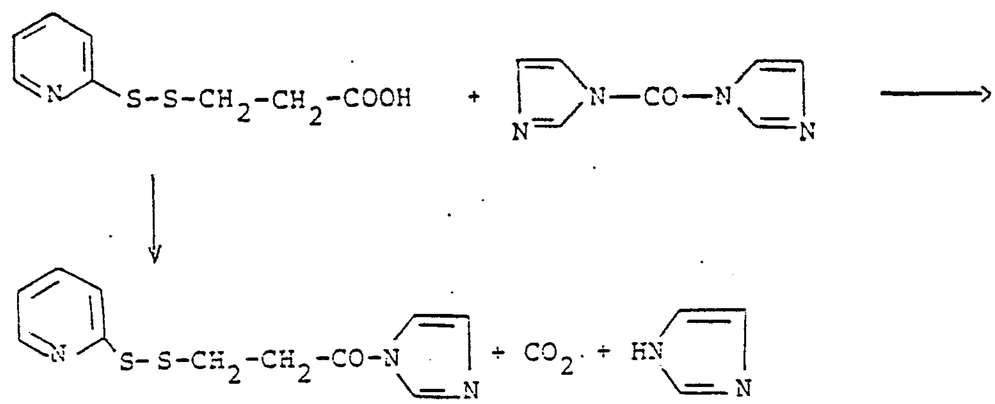

430 mg d'acide (pyridyl-2 disulfanyl)-3 propionique sont dissous dans 2 ml de THF. A cette solution sont ajoutés 405 mg de CDI. Le mélange est agité 1/4 h à 25 °C. On observe un dégagement gazeux de CO2. Le milieu réactionnel est utilisé directement et immédiatement sans purification.

B) Préparation de la chaîne A de ricine correctement fonctionnalisée

1) Blocage du thiol naturel par le N-éthylmaléimide

15 ml de solution aqueuse de chaîne A de ricine à 8 mg/ml (soit 4,1 micromoles de chaîne A) sont additionnés d'une solution aqueuse de mercapto-2-éthanol, de telle sorte que la concentration finale de ce dernier soit de 1 %.

La solution est laissée au repos une heure, puis dialysée en continu contre du tampon phosphate 125 mM pH 7 renouvelé pendant 40 heures à raison de 300 ml/h. Selon la méthode d'ELLMAN (Methods in Enzymology 25, 457, 1972), on a dosé 0,9 équivalent SH par mole de chaîne A de ricine.

On procède au blocage de cette fonction SH par le N-éthylmaléimide selon la méthode décrite dans Methods in Enzymology 11, 541 (1967). Pour cela, la chaîne A de ricine obtenue à l'étape précédente est incubée pendant 2 heures à 30 °C en présence de 20 équivalents de N-éthylmaléimide par mole de chaîne A. On élimine l'excès de réactif par dialyse en continu contre du tampon phosphate 125 mM pH 7 renouvelé pendant 20 heures à raison de 500 ml/h. On obtient ainsi 13 ml d'une solution de chaîne A de ricine à 7 mg/ml, ne présentant plus de groupements thiol dosables par le réactif d'ELLMAN. Le produit ainsi obtenu est désigné ultérieurement sous l'appellation chaîne A (NEM).

2) Modification des tyrosines

A 18 mg de chaîne A (NEM) dans 10 ml de tampon phosphate 125 mM pH 7 (soit 0,6 micromoles) sont ajoutés goutte à goutte 300 microlitres de la solution de réactif de couplage dans le THF obtenue précédemment. Le milieu réactionnel est agité 15 min. à 25 °C puis la solution est purifiée de l'excès de réactif par dialyse contre du tampon phosphate 125 mM pH 7. On obtient ainsi 9,5 ml d'une solution de chaîne A (NEM) modifiée à 1,55 mg/ml de protéine.

Par dosage spectrophotométrique à 343 nm de la pyridine thione-2 libérée par échange avec le mercapto-2 éthanol, on constate que l'on a obtenu une chaîne A (NEM) modifiée portant 1,6 groupements activateurs par mole de chaîne A (NEM).

C) Préparation de l'anticorps modifié

A 45 mg d'une solution d'anticorps T101 à 9 mg/ml, soit 0,3 micromoles, sont ajoutés 25 microlitres d'une solution d'anhydride S-acétyl mercapto succinique (SAMSA) à 170 mg/ml dans le diméthylformamide. Le milieu réactionnel est agité pendant 2 heures à 4 °C puis purifié par dialyse des réactifs contre du tampon phosphate 125 mM pH 7 pendant 24 heures à un débit de 400 ml/heure. On obtient ainsi 4,1 ml d'une solution d'anticorps modifié à 8,6 mg/ml. A cette solution d'anticorps sont ajoutés 0,46 ml d'une solution de chlorhydrate d'hydroxylamine 0,5 M. Après une incubation de 1 h à 30 °C, le milieu réactionnel est purifié par dialyse des réactifs contre le tampon phosphate 125 mM pH 7.

Par dosage spectrophotométrique des groupements —SH ainsi libérés par la méthode d'ELLMAN, on constate que l'on a obtenu un anticorps portant 5 groupements activateurs par mole d'anticorps.

D) Préparation de l'immunotoxine

A 2,1 ml de la solution d'anticorps précédemment obtenue (soit 0,11 micromole), on ajoute 5,2 ml de la solution de chaîne A modifiée (0,27 micromoles). On laisse le mélange incuber 5 heures à 30 °C.

Le milieu réactionnel est purifié sur colonne de Sephadex G100 avec mesure de la densité optique de l'effluent à 280 nm. On obtient 17 ml d'une solution d'immunotoxine à 1,1 mg/ml (soit 18,7 mg). Cette solution contient 0,32 mg/ml en chaîne A (NEM) modifiée. Le taux de couplage moyen de cette préparation est de 2 chaînes A (NEM) par mole d'anticorps.

On obtient ainsi une immunotoxine de formule V ci-dessus, où :

A' est le radical de la sous-unité A de la ricine dont les groupes SH sont bloqués par le N-éthylmaléimide

P' est le radical de l'anticorps T101

W' est un groupement de formule

$$-(NH-Y'-E')n-S-S-E-Y-Z-$$

dans laquelle

Y' est —CO—

E' est $-\underset{|}{C}H-$ $(CH_2-COOH)$

E est $-CH_2-CH_2-$

Y est —CO—

Z est —O—

n est 1.

E) Test d'activité des immunotoxines

La propriété biologique fondamentale de la chaîne A de ricine est d'inhiber la synthèse protéique dans les cellules eucaryotes par altération de la sous-unité ribosomale 60S.

Les tests réalisés sont donc des tests d'inhibition de la synthèse protéique :

soit sur un modèle acellulaire (test no 1)

soit sur un modèle cellulaire (test no 2)

1) Le modèle acellulaire (test no 1)

Le protocole in vitro utilise des fractions subcellulaires de foie de rat convenablement complémentées et capables d'incorporer la 14C-phénylalanine en présence d'un ARN messager artificiel : l'acide polyuridylique.

Le mode opératoire employé pour préparer les fractions subcellulaires et pour mesurer l'incorporation de 14C-phénylalanine est une adaptation de la méthode décrite dans Biochemica Biophysica Acta 312, 608-615 (1973), mettant en œuvre à la fois une fraction microsomale et une fraction de cytosol des hépatocytes de rat. L'échantillon contenant la chaîne A est introduit sous la forme d'une solution

convenablement diluée dans un tampon Tris HCl 50 mM, pH 7,6 contenant du mercapto-2 éthanol à 0,2 % et de la sérum albumine bovine à 15 microgrammes/ml. A partir des données de comptage, on calcule par rapport à un milieu témoin sans inhibiteur le pourcentage d'inhibition d'incorporation de 14C-phénylalanine dans les protéines pour chaque milieu réactionnel contenant de la chaîne A de ricine. L'ensemble de ces valeurs permet de déterminer la concentration de chaîne A de ricine (ou CI50) qui inhibe 50 % d'incorporation de la 14C-phénylalanine dans les conditions de l'essai.

2) Le modèle cellulaire (test no 2)

Ce test mesure l'effet des substances étudiées sur l'incorporation de 14C-leucine dans des cellules cancéreuses en culture.

Les cellules utilisées dépendent de la spécificité de l'anticorps choisi pour la fabrication de l'immunotoxine. Dans cet exemple, il s'agit de cellules de la lignée lymphoblastoïde humaine CEM portant naturellement l'antigène T65.

Ces cellules sont incubées en présence de préparations des substances à étudier puis soumises en fin d'incubation à une mesure de leur taux d'incorporation de 14C-leucine.

Cette mesure est effectuée selon une technique adaptée de la technique décrite dans Journal of Biological Chemistry 249 (11), 3557-3562 (1974) utilisant le tracteur 14C-leucine pour la détermination du taux de synthèse protéique. La détermination de la radioactivité incorporée est effectuée ici sur les cellules entières isolées par filtration.

A partir de ces déterminations, on peut tracer les courbes effets/doses présentant en abscisse la concentration des substances étudiées et en ordonnée l'incorporation de 14C-leucine exprimée en pourcentage de l'incorporation par des cellules témoins en l'absence de la substance à étudier. On peut ainsi déterminer pour chaque substance étudiée la concentration qui inhibe de 50 pour cent l'incorporation de 14C-leucine dans les cellules ou « concentration inhibitrice 50 » (CI50). On a vérifié que la mesure d'incorporation de la 14C-leucine dans les cellules entières conduit à la détermination de CI50 identiques à celles obtenues par la méthode classique de mesure de la synthèse protéique.

Résultats

a) Test no 1 (modèle acellulaire)

L'activité inhibitrice de la chaîne A (NEM) modifiée a été déterminée. La CI50 est égale à $3,6 \cdot 10^{-10}$ mole/litre. La CI50 de la chaîne A témoin de l'expérience est de $1,2 \cdot 10^{-10}$ mole/l. Il n'y a donc pas de perte d'activité significative de la chaîne A modifiée.

b) Test no 2 (modèle cellulaire)

Dans les conditions expérimentales définies ci-dessus, la CI50 en présence d'activateur (monensine 50 nM) est de $1,2 \cdot 10^{-12}$ mole/l, ce qui représente une activité $5 \cdot 10^4$ fois supérieure à celle de la chaîne A (CI50 = $6 \cdot 10^{-8}$ mole/l).

## Exemple 2

P1 = chaîne A de ricine à l'état natif.

P2 = anticorps T101 sur lequel on a introduit un groupement disulfure activé par l'intermédiaire de l'hydroxyle des tyrosines.

A) Préparation du réactif de couplage : identique à celle décrite à l'exemple 1

B) Préparation de l'anticorps modifié

A 12,8 mg d'anticorps T101 dans 2,8 ml de tampon phosphate 125 mM pH 7, sont ajoutés goutte à goutte 34 microlitres de la solution de THF diluée au 1/2 décrite précédemment (200 équivalents/mole d'IgG). Le milieu réactionnel est agité 1/4 h à température ambiante puis purifié par dialyse. On obtient ainsi 2,6 ml d'une solution d'anticorps modifié à 4,55 mg/ml.

Par dosage spectrophotométrique à 343 nm de la pyridine thione-2 libérée par échange avec le mercapto-2 éthanol, on constate que l'on a obtenu un anticorps portant 2,2 groupements activateurs par mole d'anticorps.

C) Préparation de l'immunotoxine

A 750 microlitres d'une solution de chaîne A de ricine à 7,1 mg/ml (soit 0,177 micromoles) sont ajoutés 2,5 ml d'une solution d'anticorps activé à 4,55 mg/ml (soit 0,075 micromoles d'anticorps). On laisse le mélange incuber 18 h à 25 °C. Le milieu réactionnel est purifié sur colonne de Sephadex G100

comme décrit à l'exemple 1. On obtient 13 ml d'une solution d'immunotoxine à 0,8 mg/ml (soit 10,4 mg). Cette solution contient 0,2 mg/ml de chaîne A. Le taux de couplage moyen de cette préparation est de 1,5 chaîne A par mole d'anticorps.

On obtient ainsi une immunotoxine de formule VI ci-dessus, où :
A' est le radical de la sous-unité A de la ricine
P' est le radical de l'anticorps T101
W' est un groupement de formule

—Z''—Y—E—S—S—(E'—Y'—Z')n—

dans laquelle
Z'' est —O—
Y est —CO—
E est —CH2—CH2—
n est zéro — m est 1,5

D) Tests d'activité

Test no 2 (modèle cellulaire)

Dans des conditions expérimentales identiques à celles de l'exemple 1, la CI50 en présence d'activateur (Monensine 50 nM) est de $3,5 \cdot 10^{-13}$ mole/l ce qui représente une activité cytotoxique $1,5 \cdot 10^{5}$ fois supérieure à celle de la chaîne A dans la même expérience (CI50 = $0,6 \cdot 10^{-8}$ mole/l).

**Revendications** (pour les Etats contractants : BE, CH, DE, FR, GB, IT, LI, LU, NL, SE)

1. Imidazolides de formule :

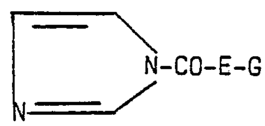

dans laquelle :
E représente un groupe —(CH2)p— où p est un nombre entier de 2 à 7 ou un groupe

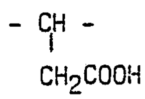
et

G est un groupement de structure —S—S—X, où X est un radical activateur choisi parmi les groupes 2-pyridyle et 4-pyridyle non substitués ou substitués par un ou des halogènes ou des radicaux alkyle contenant jusqu'à 5 atomes de carbone, carboxyle, alcoxycarbonyle dont le groupe alcoxy contient jusqu'à 5 atomes de carbone ; le groupe phényle non substitué ou substitué par un ou des halogènes ou des groupes nitro, alcoxy contenant jusqu'à 5 atomes de carbone, carboxyle ou alcoxycarbonyle dont le groupe alcoxy contient jusqu'à 5 atomes de carbone, ou un groupe alcoxycarbonyle dont le groupe alcoxy contient jusqu'à 5 atomes de carbone.

2. Utilisation des imidazolides selon la revendication 1 comme réactif de couplage des protéines.

**Revendications** (pour l'Etat contractant AT)

1. Procédé pour l'obtention d'imidazolides de formule :

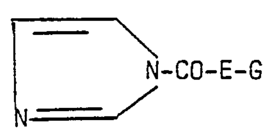

dans laquelle :
E représente un groupe —(CH2)p— où p est un nombre entier de 2 à 7 ou un groupe

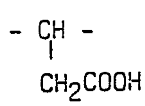
et

G est un groupement de structure —S—S—X, où X est un radical activateur choisi parmi les groupes 2-pyridyle et 4-pyridyle non substitués ou substitués par un ou des halogènes ou des radicaux alkyle contenant jusqu'à 5 atomes de carbone, carboxyle, alcoxycarbonyle dont le groupe alcoxy contient jusqu'à 5 atomes de carbone ; le groupe phényle non substitué ou substitué par un ou des halogènes ou des groupes nitro, alcoxy contenant jusqu'à 5 atomes de carbone, carboxyle ou alcoxycarbonyle dont le groupe alcoxy contient jusqu'à 5 atomes de carbone, ou un groupe alcoxycarbonyle dont le groupe alcoxy contient jusqu'à 5 atomes de carbone, caractérisé en ce qu'il consiste à faire réagir un composé de formule :

$$G—E—COOH \qquad (II)$$

dans laquelle G et E sont tels que définis ci-dessus, avec le carbonyldiimidazole de formule :

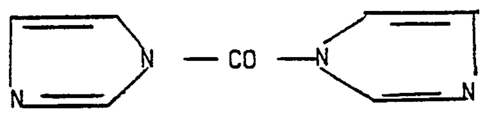

(III)

dans un solvant organique, à la température de 10 à 40 °C.

2. Utilisation des imidazolides selon la revendication 1 comme réactif de couplage de protéines.

**Claims** (for the Contracting States : BE, CH, DE, FR, GB, IT, LI, LU, NL, SE)

1. Imidazolides of the formula :

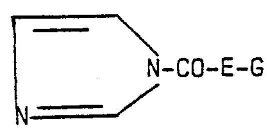

in which :

E represents a group —(CH2)p—, in which p is an integer from 2 to 7, or a group

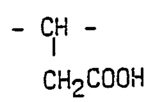

and

G is a group of the structure —S—S—X, in which X is an activating radical chosen from pyridin-2-yl and pyridin-4-yl groups which are unsubstituted or substituted by one or more halogens or alkyl radicals containing up to 5 carbon atoms, carboxyl or alkoxycarbonyl radicals, of which the alkoxy group contains up to 5 carbon atoms ; the phenyl group which is unsubstituted or substituted by one or more halogens or nitro, alkoxy groups containing up to 5 carbon atoms, carboxyl or alkoxycarbonyl groups of which the alkoxy group contains up to 5 carbon atoms, or an alkoxycarbonyl group of which the alkoxy group contains up to 5 carbon atoms.

2. Use of the imidazolides according to claim 1 as protein coupling reagent.

**Claims** (for the Contracting State AT)

1. A process for the preparation of imidazolides of the formula :

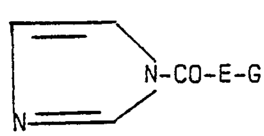

in which :

E represents a group —(CH2)p—, in which p is an integer from 2 to 7, or a group

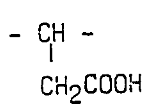

and

G is a group of the structure —S—S—X, in which X is an activating radical chosen from pyridin-2-yl and pyridin-4-yl groups which are unsubstituted or substituted by one or more halogens or alkyl radicals containing up to 5 carbon atoms, carboxyl or alkoxycarbonyl radicals, of which the alkoxy group contains up to 5 carbon atoms ; the phenyl group which is unsubstituted or substituted by one or more halogens or nitro, alkoxy groups containing up to 5 carbon atoms, carboxyl or alkoxycarbonyl groups of which the alkoxy group contains up to 5 carbon atoms, or an alkoxycarbonyl group of which the alkoxy group contains up to 5 carbon atoms, characterized in that it consists in reacting a compound of the formula :

G—E—COOH (II)

in which G and E are as defined above, with the carbonyldiimidazole of the formula :

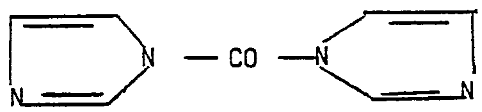

(III)

in an organic solvent at the temperature of 10 to 40 °C.

2. Use of the imidazolides according to claim 1 as a protein coupling reagent.

**Patentansprüche** (für die Vertragsstaaten : BE, CH, DE, FR, GB, IT, LI, LU, NL, SE)

1. Imidazolide der Formel :

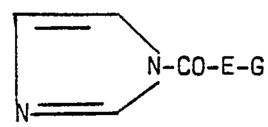

worin :

E eine Gruppe —(CH2)p—, in der p eine ganze Zahl von 2 bis 7 ist, oder eine Gruppe

- CH -
  |
$CH_2COOH$ repräsentiert und

G eine Gruppe der Struktur —S—S—X ist, wo X ein Aktivator-Radikal ist, ausgewählt aus den Gruppen 2-Pyridyl und 4-Pyridyl, nicht substituiert oder substituiert durch ein oder mehrere Halogene oder Alkyl-Radikale, die bis 5 Kohlenstoffatome aufweisen, Karboxyl-, Alkoxykarbonyl-Radikale, deren Alkoxygruppe bis zu 5 Kohlenstoffatome aufweist ; die Phenylgruppe, nicht substituiert oder substituiertdurch ein oder mehrere Halogene oder Nitro-, Alkoxygruppen, die bis 5 Kohlenstoffatome aufweisen, Karboxyl- oder Alkoxykarbonylgruppen, deren Alkoxygruppe bis 5 Kohlenstoffatome aufweist, oder eine Alkoxykarbonylgruppe, deren Alkoxygruppe bis 5 Kohlenstoffatome aufweist.

2. Verwendung der Imidazolide gemäss Anspruch 1 als Proteinkupplungsreagenz.

**Patentansprüche** (für den Vertragsstaat AT)

1. Verfahren zur Herstellung von Imidazoliden der Formel :

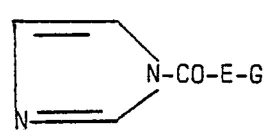

worin :

E eine Gruppe —(CH2)p—, in der p eine ganze Zahl von 2 bis 7 ist, oder eine Gruppe

- CH -
  |
$CH_2COOH$ repräsentiert und

G eine Gruppe der Struktur —S—S—X ist, wo X ein Aktivator-Radikal ist, ausgewählt aus den

Gruppen 2-Pyridyl und 4-Pyridyl, nicht substituiert oder substituiert durch ein oder mehrere Halogene oder Alkyl-Radikale, die bis 5 Kohlenstoffatome aufweisen, Karboxyl-, Alkoxykarbonyl-Radikale, deren Alkoxygruppe bis zu 5 Kohlenstoffatome aufweist ; die Phenylgruppe, nicht substituiert oder substituiert durch ein oder mehrere Halogene oder Nitro-, Alkoxygruppen, die bis 5 Kohlenstoffatome aufweisen, Karboxyl- oder Alkoxykarbonylgruppen, deren Alkoxygruppe bis 5 Kohlenstoffatome aufweist, oder eine Alkoxykarbonylgruppe, deren Alkoxygruppe bis 5 Kohlenstoffatome aufweist, dadurch gekennzeichnet, dass es darin besteht, eine Verbindung der Formel :

$$G—E—COOH \qquad (II)$$

worin G und E wie oben definiert sind, mit dem Karbonyldiimidazol der Formel :

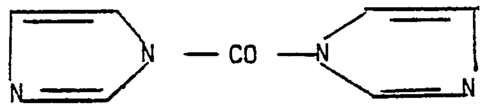

(III)

in einer organischen Lösung bei der Temperatur von 10 bis 40 °C reagieren zu lassen.

2. Verwendung der Imidazolide gemäss Anspruch 1 als Proteinkupplungsreagenz.